# EUROPEAN PATENT APPLICATION

(11) **EP 1 864 643 A1**
(43) Date of publication of application: **12.12.2007**
(21) Application number: 06730359.4
(22) Date of filing: 29.03.2006
(51) Int. Cl.: A61K 6/00

(54) **OIL/FAT-CONTAINING COMPOSITION FOR SUPPRESSION OF CANCER DEVELOPMENT**

(30) Priority: 30.03.2005 JP 2005098341
(71) Applicant: Kaneka Corporation, Kita-ku Osaka-shi, Osaka 530-8288 (JP)
(72) Inventor: KITANO, Mitsuaki, Takasago-shi, Hyogo 6760013 (JP); ARAI, Naoki, Kyoto-shi, Kyoto 6068396 (JP); IKEHARA, Toshinori, Takasago-shi, Hyogo 6760011 (JP); NAKAGAWA, Kaku, Kyoto-shi Kyoto 6038123 (JP)
(74) Representative: Vossius & Partner
(86) International application number: PCT/JP2006/306411
(87) International publication number: WO 2006/106705

(57) **Abstract**

The present invention relates to an oil/fat-containing composition for suppressing carcinogenesis, the composition including a solution produced by bringing a hydrophobic extract of licorice, which has been produced by bringing licorice into contact with an alcohol, into contact with an oil/fat. According to the present invention, there are provided an oil/fat-containing composition for prevention or treatment of the cancer, which can act through oral administration, and can be safely ingested on a day-to-day basis, pharmaceuticals and foods containing the oil/fat-containing composition.

## Description

### Technical Field

The present invention relates to an oil/fat-containing composition for suppressing carcinogenesis, which is suitable for use in preparing food and drink such as health foods and food with health claims (Food for Specified Health use and Food with Nutrient Function Claims), pharmaceuticals, quasi-drugs, cosmetics, and the like.

### Background Art

Until now, various studies have been made on cancer therapy. In particular, many studies and developments on therapeutic agents have been performed, and some drugs are actually used in the clinical field. However, a magic bullet capable of completely curing the cancer has not been discovered, and cancer therapeutic agents such as anticancer agents or immunotherapeutic agents which are now being used have problems of weak therapy effects and strong side effects. The problems of the therapeutic agents used after occurrence of the cancer are caused by difficulty in specifically affecting only on cancer cells without affecting normal cells and by intrinsic safety issues of an agent including a chemically synthesized substance.

In recent years, aside from therapeutic agents to be used after emergence of the cancer, greater importance is placed on a search for a carcinogenesis-suppressing substance from the viewpoint of suppression of the emergence of the cancer. If an effective carcinogenesis-suppressing substance can be extracted or synthesized and is used as a drug for suppressing carcinogenesis, it becomes possible to prevent, in particular, hereditary cancers or occupational cancers. The drug is considered to contribute significantly to new medical care in the future and health maintenance in daily life. Preferably, such carcinogenesis-suppressing agent should be ingested easily in daily life. In addition, the agent is required to exhibit its effect even in the case of oral administration without side effects. However, no report has been made on the use of a certain harmless carcinogenesis-suppressing substance as an agent for prevention or treatment of the cancer.

Licorice is a plant belonging to the Glycyrrhiza (Fabaceae), and is used as food or pharmaceuticals (herbal medicine), and examples of the main species of the plant include Glycyrrhiza. glabra, G. uralensis, G. inflata, and the like. All of those species commonly contain glycyrrhizin (glycyrrhizinic acid) which is a hydrophilic component, while they contain species specific compound as hydrophobic component flavonoids. The flavonoids specific to the species may be used for identification of the species of licorice.

Among extracts obtained from licorice, a hydrophobic extract of licorice, which contains a large amount of licorice flavonoid and an extremely small amount of glycyrrhizin, has been found to be useful for prevention and/or treatment of multiple risk factor syndrome (see Patent Document 1) . A recent study has further revealed that the hydrophobic extract of licorice has PPARγ ligand activity, and the active ingredient is flavonoid (see Patent Document 2). In particular, characteristic flavonoids contained in an extract derived from G. glabra, i.e., glabrene, glabridin, glabrol, 3'-hydroxyl-4'-O-methylglabridin, 4'-O-methylglabridin, and hyspaglabridin B, have been found to have high PPARγ ligand activity (see Patent Document 2).

On the other hand, a compound contained in a hydrophobic extract of licorice such as hydrophobic licorice flavonoid, does not substantially dissolve in water, and the extract obtained with an organic solvent is difficult to use because the compound deteriorates significantly with time (easily cakes and colors). In order to solve the problems, there has been proposed a method of stabilizing the compound by dissolving in a medium-chain fatty acid triglyceride before formulation (see Patent Document 3). In Patent Document 3, availability of the hydrophobic licorice flavonoid pharmaceutical as an antioxidant, antibacterial agent, enzyme inhibitor, colorant, antitumor agent, antiallergic agent, or antiviral agent is suggested, but not tested, and it is unclear whether the hydrophobic licorice flavonoid pharmaceutical can be used for such applications in actuality. Moreover, Patent Document 3 does not disclose a detailed examination on the applications and the kind of an extraction solvent to be used for obtaining a hydrophobic extract of licorice.
Patent Document 1: WO02/047699
Patent Document 2: WO03/037316
Patent Document 3: JP 2794433 A

### Disclosure of the Invention

### Problem to be solved by the Invention

An object of the present invention is to provide an oil/fat-containing composition for suppressing carcinogenesis.

### Means for solving the Problems

Surprisingly, the inventors of the present invention have found that a solution that is an oil/fat-containing composition, and that is obtained by bringing an alcohol extract, which has been obtained by bringing licorice into contact with an alcohol, into contact with oil/fat, has a carcinogenesis-suppressing effect, thus achieving the object of the present invention. Accordingly, the present invention provides the followings.

(1) An oil/fat-containing composition for suppressing carcinogenesis, including a solution obtained by bringing a hydrophobic extract of licorice into contact with oil/fat.
(2) A composition according to Item (1), including the hydrophobic extract of licorice in an amount that allows the extract to be ingested at 0. 45 to 3. 6 mg/kg body weight per adult a day.
(3) A composition according to Item (1), in which the oil/fat contains a glycerin fatty acid ester including a medium-chain fatty acid triglyceride and/or a partial glyceride comprising fatty acid.
(4) A composition according to Item (1), in which the oil/fat contains a glycerin fatty acid ester including a medium-chain fatty acid triglyceride at a concentration of 50 wt% or more.
(5) A composition according to Item (4), in which the oil/fat contains a glycerin fatty acid ester including a partial glyceride comprising fatty acid at a concentration of 50 wt% or more.
(6) A composition according to any one of Items (1) to (5), which is used as food and drink.
(7) A composition according to any one of Items (1) to (5), which is used as pharmaceuticals.
(8) A method of producing a composition for preventing carcinogenesis and/or treating cancer, which is characterized by adding a solution, obtained by bringing a hydrophobic extract of licorice into contact with oil/fat, as an effective ingredient.
(9) A method of preventing carcinogenesis and/or treating cancer, which is characterized by administering a solution, obtained by bringing a hydrophobic extract of licorice into contact with oil/fat, as an effective ingredient.

### Effects of the Invention

The present invention provides the oil/fat-containing composition for preventing or treating carcinogenesis, which is suitable for use in preparing food and drink such as health food and food with health claims (Food for Specified Health Use and Food with Nutrient Function Claims), pharmaceuticals, quasi-drugs, cosmetics, and the like.

### Best Mode for carrying out the Invention

Hereinafter, embodiments of the present invention will be described in detail.

According to one aspect of the present invention, the present invention provides an oil/fat-containing composition for preventing or treating cancer, which includes a solution obtained by bringing a hydrophobic extract of licorice, extracted from licorice with an organic solvent, into contact with oil/fat.

The oil/fat-containing composition described in this case includes: a composition directly obtained by dissolving a hydrophobic extract of licorice in oil/fat; a composition obtained by removing insoluble impurities from oil/fat in which a hydrophobic extract of licorice is dissolved; a composition obtained by adding oil/fat thereto; or a composition obtained by removing a hydrophobic extraction solvent from the composition; and a composition for food and drink, pharmaceuticals, quasi-drugs, and the like, which contains the aforementioned compositions. That is, the oil/fat-containing composition of the present invention includes: a composition obtained by dissolving a hydrophobic extract of licorice in oil/fat; a composition obtained by mixing another component to the composition; and a composition for food and drink, pharmaceuticals, quasi-drugs, and the like, which is obtained by processing the composition.

The amount of a hydrophobic extract of licorice in an oil/fat-containing composition is not particularly limited, but from the viewpoint of solubility in oil/fat, the upper limit is preferably 30 wt% or less, the amount being converted so as to be free of hydrophobic extraction solvent, while from the viewpoint of efficacy of the hydrophobic extract of licorice, the lower limit is preferably 0.01 wt% or more. More preferably, the extract is contained in an oil/fat-containing composition at a concentration of 0.1 to 30 wt%, more preferably 1 to 10 wt%.

A licorice material to be used in the present invention is not limited, as long as it is derived from a plant of the genus Glycyrrhiza (Fabaceae), and examples thereof include Glycyrrhiza. glabra, G. uralensis, and G. inflata, and the like. Of those, from the viewpoint of safety, preferable is G. glabra licorice that is most widely distributed and widely eaten.

In the present invention, a method of obtaining a hydrophobic extract of licorice is not particularly limited, and the extract can be obtained from G. glabra licorice. In the case of obtaining the extract from licorice, the method is not particularly limited, and the extract can be obtained by extraction with an organic solvent such as an alcohol, ethyl acetate, or acetone, and the like. The extraction is intended to be used as food and drink, pharmaceuticals, quasi-drugs, and the like, and therefore an extraction with an alcohol, in particular, extraction with ethanol is preferable. Ethanol is not particularly limited, as long as it can be used in production of foods and pharmaceuticals, but for the purpose of extraction of a hydrophobic component, the water content is 30% or less, preferably 10% or less, more preferably 5% or less.

The weight ratio of an alcohol and a licorice material is not particularly limited, but from the viewpoint of the extraction efficiency, the ratio is preferably 0.5:1 to 20:1, more preferably 1:1 to 10:1. Meanwhile, the extraction temperature and extraction time are not particularly limited, but for the purpose of an increase in the extraction rate, the extraction is performed at a temperature of 20°C or higher, preferably 20 to 80°C, more preferably 30 to 60°C for an extraction time of 1 hour or more, preferably 1 to 10 hours, more preferably 1 to 3 hours. If an extraction procedure is repeated more than once, a hydrophobic component can be extracted efficiently from a licorice material. Therefore, the extraction procedure is preferably repeated, at least, twice or more.

The thus-obtained extract may be used as it is, or a crudely purified or purified product obtained by column treatment, deodorization treatment, decolorization treatment, and the like, or a solid matter obtained by removing a solvent from the purified or crudely purified product may be used. As the extract, a purified product may be used, or a crudely purified product may be used as long as the extract contains no impurities inappropriate for food and drink, pharmaceuticals, quasi-drugs, and the like.

The oil/fat to be used in the present invention is a glycerin fatty acid ester including a medium-chain fatty acid triglyceride , preferably a glycerin fatty acid ester including a medium-chain fatty acid triglyceride at a concentration of about 50 wt% or more, more preferably a glycerin fatty acid ester including a medium-chain fatty acid triglyceride at a concentration of about 70 wt% or more. The medium-chain fatty acid triglyceride in this case contains a fatty acid having about 6 to 12 carbon atoms as a component fatty acid, and the component ratio of the fatty acid is not particularly limited. However, the component ratio of a fatty acid having about 8 to 10 carbon atoms is preferably about 50 wt% or more, more preferably about 70 wt% or more. In particular, a medium-chain fatty acid triglyceride with a specific gravity at about 20°C of about 0.94 to 0.96 and with a viscosity at about 20°C of about 23 to 28 cP is more preferable. In addition, the medium-chain fatty acid triglyceride may be a natural product or may be prepared by transesterification, or the like.

Meanwhile, the oil/fat to be used in the present invention is a glycerin fatty acid ester including a partial glyceride comprising fatty acid, preferably a glycerin fatty acid ester including a partial glyceride, at a concentration of about 50 wt% or more, more preferably a glycerin fatty acid ester including a partial glyceride at a concentration of about 70 wt% or more. The partial glyceride in this case is a diglyceride (1,2-diacylglycerol or 1,3-diacylglycerol) or a monoglyceride (1-monoacylglycerol or 2-monoacylglycerol), and either thereof or a mixed product thereof may be used. The component fatty acid is not particularly limited, but a diglyceride including an unsaturated fatty acid and/or a medium-chain fatty acid is preferable from the viewpoint of processability. In addition, the partial glyceride may be a natural product or may be prepared by transesterification, or the like. Moreover, the oil/fat to be used in the present invention may be a mixed product of the medium-chain fatty acid triglyceride and partial glyceride.

In the present invention, the method of obtaining an oil/fat-containing composition is not particularly limited, and the composition may be obtained by dissolving the hydrophobic extract of licorice in oil/fat. In this case, a general operation such as stirring or mixing can be performed, but it is desirable to remove impurities insoluble in oil/fat by an operation such as filtration or centrifugation, after dissolving the extract in oil/fat by a general operation such as stirring or mixing. Alternatively, the method disclosed in WO 03/84556 is preferred. For example, the composition can be extracted directly from a licorice raw material with oil/fat, and can also be obtained by mixing oil/fat with an organic solvent, preferably ethanol, containing a hydrophobic extract of licorice, and then removing the ethanol. Moreover, in the present invention, oil/fat may be further added to a mixture of oil/fat and a hydrophobic extract of licorice, to adjust the composition ratio of them.

Usually, the hydrophobic extract of licorice in the form of powder is unstable, and its stability cannot be improved even if the extract is dissolved in an organic solvent such as ethanol. However, the stability can be improved by dissolving the extract in oil/fat to be used in the present invention. In addition, the extract has poor water solubility, and therefore if the extract is dissolved in oil/fat to be used in the present invention, it is expected to improve the property of being absorbed.

A composition of the present invention may contain a carrier, acceptable for food and drink, pharmaceuticals, or cosmetics. The carrier for pharmaceuticals may be any inactive, organic, or inorganic material suitable for, for example, oral, enteral, percutaneous, subcutaneous, or nonenteral administration, and examples thereof include, but not limited to, water, gelatin, gum arabic, lactose, microcrystalline cellulose, starch, sodium starch glycolate, calcium hydrogen phosphate, magnesium stearate, talc, and colloidal silicon dioxide, and the like. The composition may further contain another pharmacologically active agent and a general additive such as stabilizer, wetting agent, emulsifier, flavoring agent, and buffer.

An oil/fat-containing composition for preventing or treating cancer of the present invention is characterized by containing oil/fat in which at least one compound selected from the group consisting of glabrene, glabridin, glabrol, 3'-hydroxyl-4'-O-methylglabridin, 4'-O-methylglabridin, and hyspaglabridin B is dissolved.

In the present invention, the origins of glabrene, glabridin, glabrol, 3'-hydroxyl-4'-O-methylglabridin, 4'-O-methylglabridin, and hyspaglabridin B are not particularly limited, and they can be obtained from G. glabra licorice. When the compounds are obtained from licorice, a method of obtaining the compounds from licorice is not particularly limited, and the compounds are contained in a hydrophobic extract of licorice obtained by extraction with an organic solvent such as ethanol, ethyl acetate, or acetone. The extract may be used as it is, or a crudely purified or purified product, obtained by column treatment, deodorization treatment, decolorization treatment, and the like, may be used.

Naturally, any of the compounds derived from natural sources such as other plants, chemically synthesized compounds and biosynthesized compounds using cultured cells may be used. Meanwhile, as the compounds, purified products may be used, or crudely purified products may be used as long as they contain no impurities inappropriate for food and drink, pharmaceuticals, quasi-drugs, cosmetics, and the like.

Among the compounds, an oil/fat-containing composition for preventing or treating cancer of the present invention preferably contains glabridin and at least one compound selected from the group consisting of glabrene, glabrol, 3'-hydroxyl-4'-O-methylglabridin, 4'-O-methylglabridin, and hyspaglabridin B. Moreover, the composition more preferably contains glabridin, glabrene, and at least one compound selected from the group consisting of glabrol, 3'-hydroxyl-4'-O-methylglabridin, 4'-O-methylglabridin, and hyspaglabridin B. In particular, the composition most preferably contains all the following compounds: glabrene, glabridin, glabrol, 3'-hydroxyl-4'-O-methylglabridin, 4'-O-methylglabridin, and hyspaglabridin B.

Glabridin, 3'-hydroxyl-4'-O-methylglabridin, 4'-O-methylglabridin, and hyspaglabridin B are flavonoids classified into isoflavans and are compounds represented by the following formula (1).

[Chem. 1]

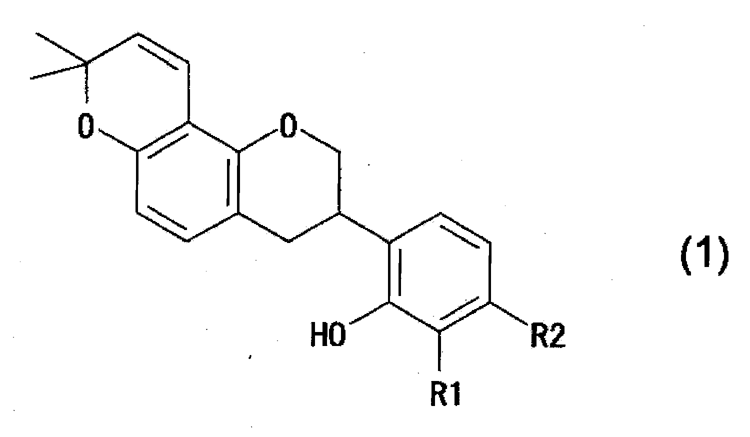

where six-membered rings are formed with the following functional groups: glabridin, R1=H, R2=OH; 3'-hydroxyl-4'-O-methylglabridin, R1=OH, R2=OCH₃; 4'-O-methylglabridin, R1=H, R2=OCH₃; and hyspaglabridin B: R1 and R2=-CH=CH-C(CH₃)₂-O-. Glabrene is a flavonoid classified into isoflav-3-enes and is a compound represented by the following formula (2).

[Chem. 2]

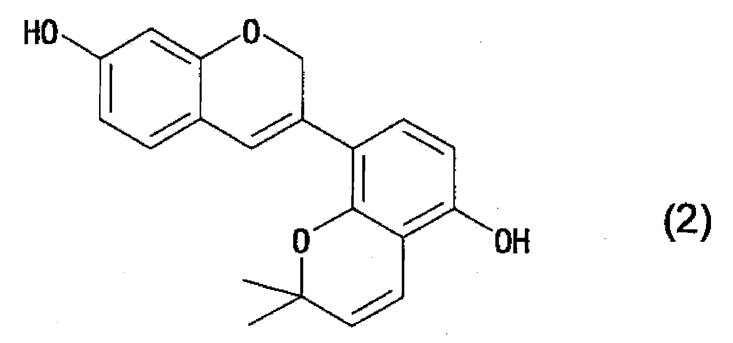

Glabrol is a flavonoid classified into flavanones and is a compound represented by the following formula (3).

[Chem. 3]

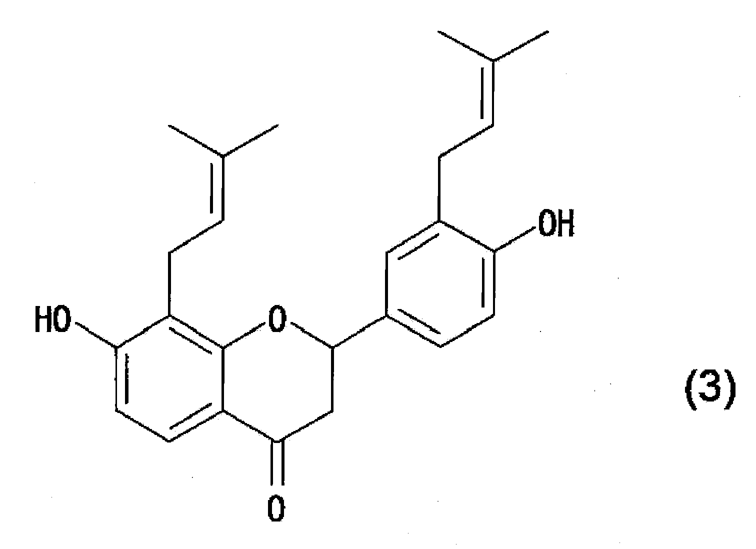

Glabrene, glabridin, glabrol, 3'-hydroxyl-4'-O-methylglabridin, 4'-O-methylglabridin, and hyspaglabridin B are components that are contained specifically in licorice, in particular, in Glycyrrhiza glabra, and are hardly contained in other plants. However, a hybrid between G. glabra and another species may contain the compounds in some cases. The compounds can be separated from other flavonoid components, and detected/quantified by an HPLC analysis using a reverse-phase column such as ODS.

An oil/fat-containing composition can be produced by adding another carrier to the resultant oil/fat extract, if necessary.

In the case of using an extract containing the compounds or crudely purified products of the compounds, the extract contains impurities other than the compounds, so it is desirable to remove impurities insoluble in oil/fat by an operation such as filtration or centrifugation, after dissolving the compounds in oil/fat by a general operation such as stirring or mixing. In the case of using purified products of the compounds, a uniform solution can be obtained easily. Meanwhile, in the case of using an extract containing the compounds or crudely purified products of the compounds, it is possible to dissolve the compounds in an organic solvent such as ethanol in advance, mix the solution with oil/fat, and distill off the organic solvent.

In the present invention, a method of dissolving glabrene, glabridin, glabrol, 3'-hydroxyl-4'-O-methylglabridin, 4'-O-methylglabridin, and hyspaglabridin B in oil/fat is not particularly limited, and it can be performed by a general operation such as stirring or mixing.

In general, glabrene, glabridin, glabrol, 3'-hydroxyl-4'-O-methylglabridin, 4'-O-methylglabridin, and hyspaglabridin B in the forms of powder are unstable, and even if the compounds are dissolved in an organic solvent such as ethanol, the stability cannot be improved. However, when the compounds are dissolved in oil/fat to be used in the present invention, the stability can be improved. The oil/fat are the same as those of the above-mentioned embodiment.

The form of an oil/fat-containing composition for preventing or treating cancer of the present invention is not particularly limited, and the composition is suitable for use in preparing food and drink such as health food and food with health claims (Food for Specified Health Use and Food with Nutrient Function Claims), pharmaceuticals, quasi-drugs, cosmetics, and the like. For example, oil/fat, in which at least one compounds selected from the group consisting of glabrene, glabridin, glabrol, 3'-hydroxyl-4'-O-methylglabridin, 4'-O-methylglabridin, and hyspaglabridin B is dissolved, can be used singly as it is for cooking, soft capsule preparations, lotions, or the like. In addition, the composition can be freely blended with an oily object substance, so it can be blended with other oil/fat to adjust the physical properties according to the purposes. In this case, the other oil/fat is preferably food or pharmaceuticals. The kinds and used amounts of the oil/fat may be determined in view of various conditions such as physical properties and operating temperature ranges required for each product, and the characteristics, such as consistency and melting point, can be controlled by optimizing the kinds and used amounts. Examples of the oil/fat include: vegetable oils such as corn oil, rapeseed oil, high erucin rapeseed oil, soybean oil, olive oil, safflower oil, cottonseed oil, sunflower oil, rice bran oil, palm oil, or palm kernel oil; animal oils such as fish oil, beef tallow, lard, milk fat, and yolk oil; oil/fat obtained from such materials by separation, hydrogenation, transesterification, or the like; and mixed oils thereof.

The thus-obtained oil/fat-containing composition can be used as liquid oil/fat such as cooking oil or frying oil, plastic oil/fat such as margarine or shortening, or can be used in a water-in-oil emulsion or an oil-in-water emulsion. Meanwhile, examples of an oil/fat-containing composition for food and drink produced using them as materials include: confectioneries such as chewing gum, chocolates, candies, jelly, biscuits, and crackers; frozen desserts such as ice cream and ice candy; beverages such as milk beverages, soft drinks, nutrition supplement drinks, and beauty drinks; noodles such as Japanese wheat noodles, Chinese noodles, spaghetti, and instant noodles; fish paste products such as kamaboko (fish cake), chikuwa (tubular fish cake), and hanpen (soft white fish cake); flavoring materials such as dressings, mayonnaise, and sauce; and other foods such as bread, ham, soup, various types of retort pouch food, and various types of frozen food, and the composition may also be used for pet foods, feedstuff, and the like.

Further, for the purpose of nutritional enrichment, various vitamins such as A, D, and E may be incorporated into or used in combination with the composition. As taste enhancers, various salts, various flavors, and milk-related substances such as whole milk powder, skim milk powder, fermented milk, and milk fat may also be incorporated into or used in combination with composition. Meanwhile, in addition to the above-mentioned materials, all of antioxidants, colorants, and the like to be used in general water-in-oil emulsions or oil-in-water emulsions may be used.

In order to exert the effects of preventing and/or suppressing carcinogenesis by glabrene, glabridin, glabrol, 3'-hydroxyl-4'-O-methylglabridin; 4'-O-methylglabridin, and hyspaglabridin B, contained in the oil/fat-containing composition for preventing and/or suppressing carcinogenesis of the present invention, the oil/fat-containing composition desirably contains the compounds within a licorice extract, in an amount that allows the extract to be ingested at 0.01 to 10 mg/kg body weight per adult a day, preferably 0.45 to 3.6 mg/kg body weight per adult a day. Note that the contents of the compounds were measured by an HPLC analysis using a reverse-phase column such as ODS.

The term "suppressing carcinogenesis" as used herein refers to suppression of generation of cancer cells and suppression of proliferation of generated cancer cells. The kind of a tumor suppressed by a carcinogenesis-suppressing agent of the present invention is not limited. That is, the tumor may be an epithelial tumor or nonepithelial tumor, and may be benign or malignant. For example, the tumor may be an epithelial tumor or nonepithelial tumor developed in various body organs such as stomach, intestines, lung, liver, kidney, pancreas, gallbladder, uterus, ovary, testis, prostate gland, or brain, or may be a nervous system tumor, a bone tumor, a lymphoid tumor, and the like.

Examples of administration of a carcinogenesis-suppressing agent of the present invention or ingestion of the agent as food and drink include administration for prevention of a hereditary cancer, prevention of a cancer attributed to environments or lifestyles, prevention of recurrence and metastasis after surgical removal of the tumor, or administration as one of cancer treatments. More specifically, examples thereof include; prophylactic ingestion of a carcinogenesis-suppressing agent of the present invention before incidence of cancer by a person who is considered to be likely to develop cancer according to a genetic testing; prophylactic ingestion of the agent by a person who has smoking habit or a person engaged in a work exposed to radioactive materials; and administration of the agent instead of or parallel to the administration of an anticancer agent for chemotherapy.

### Examples

Hereinafter, the present invention will be described in more detail by way of examples, but the present invention is not limited to the examples.

### <Example 1>

Afghan-grown licorice (G. glabra, 9.85 kg) was used to perform ethanol extraction (49.25 L, 45°C, 2 hours, extracted twice), and the extract was concentrated to obtain 4.4 L of a concentrated solution. Further, 3 L of the solution was concentrated, and the concentrate was treated with activated carbon and concentrated, to thereby obtain 811.2 g of an ethanol solution containing a hydrophobic extract of licorice.

Then, 629.2 g of the ethanol solution (containing 125.8 g of a hydrophobic extract of licorice) and 187.6 g of a medium-chain fatty acid triglyceride (MCT), i.e., Actor M-2 (Riken Vitamin Co., Ltd., fatty acid composition: C8:C10 = 99:1) were mixed, and the mixture was stirred while maintaining the temperature at about 80°C for about 1 hour, followed by vacuum concentration to remove ethanol. Insoluble fraction was separated by suction filtration, and 45.7 g of MCT was further added to the filtrate to thereby yield 297.0 g of an MCT solution. The resultant MCT solution (1 g) was dissolved in methanol for HPLC, and the total volume was adjusted to 100 ml. The solution was used as a sample to perform an HPLC analysis under the conditions described below, and as a result, 1 g of the MCT solution was found to contain glabrene, glabridin, glabrol, and 4'-O-methylglabridin in amounts of 6.6 mg, 30.8 mg, 12.6 mg, and 3.7 mg, respectively.

The licorice extract containing glabrene, glabridin, glabrol, and 4'-O-methylglabridin had a weight of about 30 wt% of the MCT solution.

### HPLC analysis conditions

For an analysis column, J' sphere ODS-H80, 4.6 x 250 mm (YMC), was used at a column temperature of 40°C. A mobile phase was used at a flow rate of 1 ml/min under the following gradient conditions: the percentage of acetonitrile to an aqueous 20 mM phosphate solution was maintained constant at 35% for 20 minutes from the start of the analysis, and after 20 minutes, increased at a constant rate so that the percentage reached 70% at 75 minutes later, and maintained constant at 80% from 75 minutes to 80 minutes later. The injection volume was 20 µl, and the detection was performed at a wavelength of 254 nm. The retention time for each compound, that is, glabrene, glabridin, glabrol, and 4'-O-methylglabridin was 40.0 minutes, 50.2 minutes, 58.3 minutes, and 66.8 minutes, respectively.

### <Example 2> Carcinogenesis-suppressing effect

In order to examine the effect of the MCT solution of Example 1 on carcinogenesis, a search using a liver medium-term bioassay (Ito test) was performed. This test method is one of the two-step carcinogenesis models using rodents and is employed for rat liver. This test method has been used to search for 313 or more chemical substances, and is considered to be a reliable and useful method of searching for carcinogens or carcinogenesis-suppressing substances. Therefore, this test is accepted as an alternative to a conventional long-term carcinogenicity test in the Japan/US/EU Trilateral International Conference on Harmonization of Technical Requirements for Registration of Pharmaceuticals for Human Use (ICH). Moreover, this test was performed in accordance with the good laboratory practice (GLP) standards based on "The Ordinance on Standard for Conduct of Non-Clinical Studies on Safety of Drugs" (Ordinance No. 21 of the Ministry of Health and Welfare, Japan, March 26, 1997), and the test results are highly reliable.

One hundred 6-week-old F344 male rats were divided into 7 groups (Groups 1 to 5: 15 rats, Groups 6 and 7: 9 rats) based on the randomized block design using a computer so that the average body weights showed no statistically significant differences. Diethylnitrosamine (DEN) was administered intraperitoneally to the rats once at a dose of 200 mg/kg for the purpose of an initiation treatment for liver carcinogenesis, and from two weeks after the administration, a test substance, i.e., the MCT solution was forcibly administered orally at doses of 0 (control group), 150, 300, and 600 mg/kg once a day seven times/week for six weeks (Groups 1 to 4). The doses of the MCT solution used in this test (150, 300, and 600 mg/kg) correspond to 45, 90, and 180 mg/kg body weight in terms of a licorice extract serving as an effective ingredient. In addition, phenobarbital sodium salt (S. PB) was administered with a feed to a positive control group at a concentration of 500 ppm (Group 5) . Note that a control group without the DEN treatment and a 600 mg/kg test substance group (Groups 6 and 7) were provided, too.

After a lapse of the third week of the experiment (one week after the start of test substance administration), partial hepatectomy was performed for all the animals, and lesions in the livers were amplified. After a lapse of eight weeks from the start of the experiment (after the test substance administration period), all the rats were killed to perform autopsy, and the livers were removed. Three tissue pieces were cut out of each liver and fixed with a 10% buffered formalin solution, then paraffin blocks were prepared and sliced to yield tissue specimens. The resultant rat liver tissue specimens were examined for the expression of placental glutathione S-transferase (GST-P), which is a marker of a liver precancerous lesion, by immunohistochemical staining using the avidin-biotin complex method. The areas of the liver sections and the numbers and areas of GST-P-positive cell foci with diameters of 0.2 mm or more were measured using a pathological specimen image analyzer IPAP-WIN (Sumika Technoservice Corporation), and then the areas and numbers per cm² of the liver sections were calculated to perform a quantitative analysis. Meanwhile, for the three each cases from Groups 6 and 7, a part of the residual liver subjected to the immunohistochemical test was collected, fixed with 4% glutaraldehyde, and subjected to an electron microscopical examination.

With regard to a statistical analysis, differences in body weights, organ weights, and mean values of the immunohistochemical test for the livers between Group 1 and Groups 2 to 4, were examined by a test of homogeneity of variance by a Bertlett's test at a significant level of 5%. In the case of homogeneity, a one-tailed test by a parametric Dunnett's test was performed, while in the case of non-homogeneity, a one-tailed test by a nonparametric Steel's test was performed.

Note that difference in mean values between Group 1 (control group) and Group 5 (S. PB group), which were with the DEN treatment, was tested by F-test. Difference in mean values between Group 6 (control group) and Group 7 (600 mg/kg group), which were without the DEN treatment, was tested by F-test, too. In the case of homogeneity, the student's t-test (one-tailed) was performed, while in the case of non-homogeneity, a Welch's test (one-tailed) was performed. The results of the liver weights and quantitative analyses of GST-P-positive cell foci are shown in Tables 1 and 2.

During the administration period, changes in general status, dead animals, changes in feed consumption and body weights due to the test substance administration were not observed. The water consumptions of the 300 and 600 mg/kg groups with the DEN treatment (Groups 3 and 4) and the 600 mg/kg without the DEN treatment (Group 7) were kept at high levels, and thus this was considered to be caused by administration of the test substance. The absolute weights and relative weights of the livers of the 300 and 600 mg/kg groups with the DEN treatment (Groups 3 and 4) were found to be at significantly high levels, and the relative weights of the livers of the 150 mg/kg group (Group 2) were found to be at significantly high levels. In addition, the absolute weights and relative weights of the livers of the 600 mg/kg group without the DEN treatment (Group 7) were also found to be at significantly high levels, and this was considered to be caused by administration of the test substance. However, the degrees were small, and there was no histological difference at the light microscope level. Moreover, in the electron microscopical examination of the livers performed for the 600 mg/kg group without the DEN treatment (Group 7), no changes were observed. Therefore, an increase in liver weight was not concluded to be of toxic nature. The numbers of the GST-P-positive cell foci per unit area in the livers of the 600 mg/kg group with the DEN treatment (Group 4) were found to be at significantly lower levels than those of the control group (Group 1), while the areas per unit area in the livers of the 150 and 600 mg/kg groups (Groups 2 and 4) were found to be at significantly low levels.

On the other hand, measurement of the GST-P-positive cell foci of the positive control, i.e., the S. PB group (Group 5) revealed that the number and area per unit area were found to be at evidently high levels, thereby proving the validity of this test. Note that a compound showing peroxisome proliferation in the liver is known to have false-negative or false-suppression effect in this test system. However, as described above, peroxisome proliferation in hepatocyte was not observed in the electron microscopical examination of the livers performed for the 600 mg/kg group without the DEN treatment (Group 7), and therefore the results of this test were found to include no false-negative or false-suppression effect.

As can be seen, in all the cases where the MCT solution was forcibly administered orally at doses of 150, 300, and 600 mg/kg, the numbers and areas of GST-P-positive cell foci were not increased, and they were decreased in the case of 600 mg/kg. Therefore, the MCT solution was found to have carcinogenesis-suppressing effect in rat livers instead of carcinogenesis-promoting effect.

The sensitivity of human is known to be higher (about 50 to 100-fold) than that of rat. Therefore, based on the fact that the MCT solution has carcinogenesis-suppressing effect on rats at doses of 150 to 600 mg/kg (that is, 45 to 180 mg/kg in terms of a licorice extract serving as an effective ingredient), the MCT solution is estimated to have carcinogenesis-suppressing effect on human at doses of 0.45 to 3.6 mg/kg in terms of a licorice extract.

[Table 1]

**[Table 1]**

| Sex Group | | Treatment | | Dose | Number of search | Liver weight | |
|---|---|---|---|---|---|---|---|
| | | DEN | Test substance | (mg/kg) | | Absolute weight (g) | Relative weight (%) |
| Male | 1 | + | MCT solution | 0 | 15 | 6.7311 ± 0.3642 | 2.5378 ± 0.0454 |
| | 2 | + | MCT solution | 150 | 15 | 7.0276 ± 0.4059 | 2.6255 ± 0.0946** |
| | 3 | + | MCT solution | 300 | 15 | 7.4460 ± 0.5865** | 2.7456 ± 0.2559** |
| | 4 | + | MCT solution | 600 | 14 | 7.5464 ± 0.4357** | 2.8291 ± 0.0714** |
| | 5 | + | Phenobarbital Sodium | 500 ^{a)} | 15 | 9.2781 ± 0.4913** | 3.3527 ± 0.1032** |
| | 6 | - | MCT solution | 0 | 9 | 7.6091 ± 0.5606 | 2.6264 ± 0.0989 |
| | 7 | - | MCT solution | 600 | 9 | 8.1869 ± 0.5380# | 2.8647 ± 0.1065## |

| | | | | | | | |
|---|---|---|---|---|---|---|---|
| a) : ppm **: There is a significant difference from the control group (Group 1) (P<0.01) #, ##: There is a significant difference from the control group (Group 6) (P<0.05, 0.01) | | | | | | | |

[Table 2]

**[Table 2]**

| Group | Treatment | | Dose | Number of search | GST-P-positive cell foci | |
|---|---|---|---|---|---|---|
| | DEN | Test substance | (mg/kg) | | Number (No. cm²) | Area (mm²/cm²) |
| 1 | + | MCT solution | 0 | 15 | 3.763 ± 1.503 | 0.277 ± 0.159 |
| 2 | + | MCT solution | 150 | 15 | 2.779 ± 1.228 | 0.164 ± 0.078* |
| 3 | + | MCT solution | 300 | 15 | 3.083 ± 1.287 | 0.175 ± 0.076 |
| 4 | + | MCT solution | 600 | 14 | 2.484 ± 1.054* | 0.134 ± 0.055* |
| 5 | + | Phenobarbital Sodium | 500 ^{a)} | 15 | 8.328 ± 2.467** | 0.541 ± 0.160** |
| 6 | - | MCT solution | 0 | 9 | 0.000 ± 0.000 | 0.000 ± 0.000 |
| 7 | - | MCT solution | 600 | 9 | 0.000 ± 0.000 | 0.000 ± 0.000 |

| | | | | | | |
|---|---|---|---|---|---|---|
| a): ppm *, **: There is a significant difference from the control group (Group 1) (P<0.05, 0.01). | | | | | | |

### <Example 3> Production of soft capsule

The MCT solution of Example 1 was pressed into a gelatin film using a rotary soft capsule production device to thereby yield a 350-mg soft capsule.

### <Example 4> Production of margarine

To 80 parts by weight of hydrogenated cottonseed oil (product name: SUNOU RAITO (Snow Light), Kaneka Corporation), 20 parts by weight of the MCT solution of Example 1, and 10 parts by weight of unsalted butter (Yotsuba Inc.), 0.2 parts by weight of a glycerin mono fatty acid ester (product name: EMARUJII-MS (Emulgy-MS), Riken Vitamin Co., Ltd.) and 0.2 parts by weight of lecitin were added, followed by dissolution while heating to 60°C, to thereby prepare an oil phase.

To 84.9 parts by weight of the resultant oil phase, 15.1 parts by weight of water was added to perform emulsification for 20 minutes, and the mixture was cooled and stirred using a combinator, to thereby prepare margarine.

### <Example 5> Production of concentrated milk

Ten parts by weight of the MCT solution of Example 1 was heated to 70°C, and 0.1 parts by weight of lecitin and 0.1 parts by weight of polyglycerin fatty acid ester were sequentially dissolved in the MCT solution to prepare an oil phase.

Twenty-five parts by weight of skim milk powder, 0.1 parts by weight of glycerin fatty acid ester, and, 0.1 parts by weight of sucrose fatty acid ester were dissolved in 64.6 parts by weight of water at 60°C to prepare an aqueous phase.

The resultant aqueous phase and oil phase were pre-emulsified and then sterilized using a UHT sterilization machine at 145°C for 4 seconds. Subsequently, the emulsion was subjected to vacuum cooling and then homogenized using a homogenizer at a pressure of 10 Mpa, further followed by plate cooling to 10°C, to thereby yield concentrated milk for processing.

### <Example 6> Production of mayonnaise

Ten parts by weight of brewed vinegar, 1 part by weight of salt, 0.6 parts by weight of sugar, 0.2 parts by weight of mustard powder, and 0.2 parts by weight of sodium glutamate were added to a mixing machine, and were stirred and mixed at 15 to 20°C, to thereby prepare an aqueous phase. Thereafter, 10 parts by weight of egg yolk was added to 68 parts by weight of refined oil of rice and 10 parts by weight of the MCT solution of Example 1, to perform emulsification while stirring, to thereby yield an emulsified liquid (10 to 15°C). The emulsified liquid was added by portions at 15 to 20°C while being stirred to perform pre-emulsification. Subsequently, emulsification was finished by using a colloid mill, to thereby yield mayonnaise.

## Claims

1. An oil/fat-containing composition for suppressing carcinogenesis, comprising a solution obtainable by bringing a hydrophobic extract of licorice into contact with oil/fat.

2. A composition according to Claim 1, comprising the hydrophobic extract of licorice in an amount that allows the hydrophobic extract to be ingested at 0.45 to 3.6 mg/kg body weight per adult a day.

3. A composition according to Claim 1, wherein the oil/fat contains a glycerin fatty acid ester including a medium-chain fatty acid triglyceride and/or a partial glyceride comprising fatty acid.

4. A composition according to Claim 1, wherein the oil/fat contains a glycerin fatty acid ester including a medium-chain fatty acid triglyceride at a concentration of 50 wt% or more.

5. A composition according to Claim 4, wherein the oil/fat contains a glycerin fatty acid ester including a partial glyceride comprising fatty acid at a concentration of 50 wt% or more.

6. A composition according to any one of Claims 1 to 5, which is used as food and drink.

7. A composition according to any one of Claims 1 to 5, which is used as pharmaceuticals.

8. A method of producing a composition for preventing carcinogenesis and/or treating cancer, which is **characterized by** adding a solution obtainable by bringing a hydrophobic extract of licorice into contact with oil/fat as an effective ingredient.

9. A method of preventing carcinogenesis and/or treating cancer, which is **characterized by** administering a solution obtainable by bringing a hydrophobic extract of licorice into contact with oil/fat as an effective ingredient.
